# EUROPEAN PATENT SPECIFICATION

(11) **EP 4 108 284 B1**
(45) Date of publication and mention of the grant of the patent: **18.06.2025**
(21) Application number: 20919670.8
(22) Date of filing: 24.11.2020
(51) Int. Cl.: A61M 25/09, A61B 1/01

(54) **GUIDE WIRE**
FÜHRUNGSDRAHT
FIL-GUIDE

(30) Priority: 19.02.2020 JP 2020025802
(43) Date of publication of application: 28.12.2022
(73) Proprietor: Asahi Intecc Co., Ltd., Seto-shi, Aichi 489-0071 (JP)
(72) Inventor: NAKANISHI Yuta, Seto-shi, Aichi 489-0071 (JP); KOSUGI Tomoki, Seto-shi, Aichi 489-0071 (JP); NOGUCHI Naoki, Seto-shi, Aichi 489-0071 (JP); GOTO Kenta, Seto-shi, Aichi 489-0071 (JP)
(74) Representative: Prinz & Partner mbB
(86) International application number: PCT/JP2020/043586
(87) International publication number: WO 2021/166355

(56) References cited:
- EP-A1- 2 311 518
- WO-A1-2019/155828
- JP-A- 2004 290 466
- JP-A- 2008 264 498
- JP-A- 2015 065 986
- US-A- 5 941 706
- US-A1- 2011 021 951

## Description

### TECHNICAL FIELD

The present invention relates to a guide wire.

### BACKGROUND ART

Conventionally, a guide wire including a resin marker (visibility marker) provided on an outer surface of a wire main body is known in order to observe a position or an orientation of a distal end of the guide wire inserted into a living lumen with an endoscopic camera (see Patent Literature 1, for example). For example, Patent Literature 1 discloses a guide wire provided with a raised portion forming layer containing a resin and a pigment, the raised portion forming layer functioning as a visual recognition marker on an outer surface of the guide wire.

### CITATION LIST

### Patent Literature

Patent Literature 1: JP 5509276
Patent Literature 2 : JP 2008 264498
Patent Literature 3 : WO 2019 155828
Patent Literature 4 : EP 2 311 518

### SUMMARY OF INVENTION

### TECHNICAL PROBLEM

However, the guide wire described in Patent Literature 1 has a problem in that an outer diameter of a portion of the guide wire where the marker is provided is larger than an outer diameter of a portion where the marker is not provided, and thus, there is a limit on a size of a catheter that can be used together with the guide wire. Another problem is that the marker protrudes radially outward from the resin portion, and thus, when contacting a device used together with the guide wire or an inner wall of a body cavity, the marker peels off. Still another problem is that if the outer diameter of the wire main body is reduced to suppress an increase in the outer diameter of the guide wire, the rigidity of the guide wire is reduced.

An object of the present invention is to suppress an increase of an outer diameter of a guide wire due to provision of a marker on the guide wire in a guide wire provided with a marker containing a resin on an outer surface of a core shaft.

### SOLUTION TO PROBLEM

The present invention has been made to solve at least one of the above-described problems, and can be realized as the following aspects.
(1) According to the present invention, a guide wire as defined in claim 1 is provided. The guide wire includes a core shaft having an elongated outer shape, and a coating film formed on an outer surface of the core shaft, the coating film containing a resin, in which the coating film includes a marker portion containing a pigment and being linearly represented on an outer surface of the coating film and a resin portion that does not contain the pigment. On the outer surface of the coating film, the marker portion and the resin portion are represented alternately in a stretching direction. A thickness of a portion of the coating film where the marker portion is represented on the outer surface and a thickness of a portion of the coating film where the resin portion is represented on the outer surface are substantially the same, or the thickness of the portion of the coating film where the marker portion is represented is smaller than the thickness of the portion of the coating film where the resin portion is represented.
   According to such a configuration, the thickness of the portion of the coating film where the marker portion is represented on the outer surface and the thickness of the portion of the coating film where the resin portion is represented on the outer surface are substantially the same, or the thickness of the portion of the coating film where the marker portion is represented on the outer surface is smaller than the thickness of the portion of the coating film where the resin portion is represented on the outer surface, and thus, when the guide wire is provided with the marker, it is possible to suppress an increase of an outer diameter of the guide wire.
(2) In this guide wire, in a longitudinal section along a stretching direction of the core shaft, a cross-sectional width of the marker portion decreases toward an inside of the core shaft in a radial direction. According to such a configuration, as compared to a case where the cross-sectional width of the marker portion is substantially constant toward the inside of the core shaft in the radial direction, it is possible to increase a contact area between the marker portion and the resin portion, and thus, it is possible to decrease a possibility that the marker portion and the resin portion peel of.
(3) In the guide wire of the above aspect, the marker portion may include a first region including the outer surface of the marker portion and a second region provided around the first region, and a content of the pigment in the second region may be smaller than a content of the pigment in the first region. According to such a configuration, when the pigment content of the marker portion contacting the resin portion is reduced, it is possible to maintain adhesion between the marker portion and the resin portion and it is possible to decrease a possibility that the marker portion and the resin portion peel off.
(4) In the guide wire of the above aspect, an average particle size of the pigment contained in the marker portion may be smaller than an average particle size of the resin portion. According to such a configuration, even if the resin portion is formed further to the distal side than the marker portion on the outer surface of the core shaft, when the pigment is heated on the resin portion, the pigment can impregnate the resin portion to form the marker portion.

It is noted that the present invention can be realized in various aspects, and may be realized in a mode such as a method of manufacturing a catheter, an endoscope, an image generation device, an inspection device, a treatment system, and a guide wire, for example. Several embodiments are defined in the dependent claims.

### BRIEF DESCRIPTION OF DRAWINGS

Fig. 1 is an explanatory diagram illustrating an entire configuration of a guide wire according to a first embodiment.
Fig. 2 is an explanatory diagram illustrating a cross-sectional configuration of the guide wire according to the first embodiment.
Fig. 3 is an explanatory diagram obtained when the detail X of Fig. 2 is enlarged.
Fig. 4 is an explanatory diagram illustrating a method, which does not form part of the invention, of manufacturing the guide wire according to the first embodiment.
Fig. 5 is an explanatory diagram illustrating a cross-sectional configuration of a guide wire according to a second embodiment.
Fig. 6 is an explanatory diagram illustrating a cross-sectional configuration of a guide wire according to a third embodiment.
Fig. 7 is an explanatory diagram illustrating a cross-sectional configuration of a guide wire according to a fourth embodiment.
Fig. 8 is an explanatory diagram illustrating a modification of the guide wire according to the first to fourth embodiments.

### EMBODIMENTS OF THE INVENTION

### <First Embodiment>

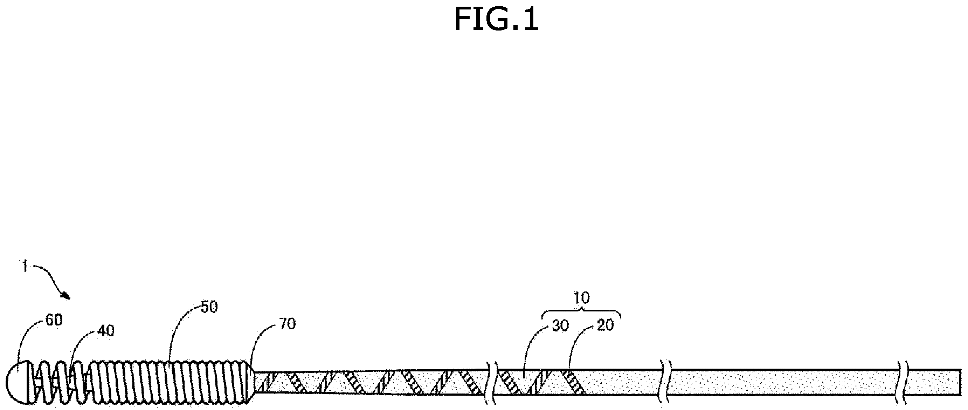

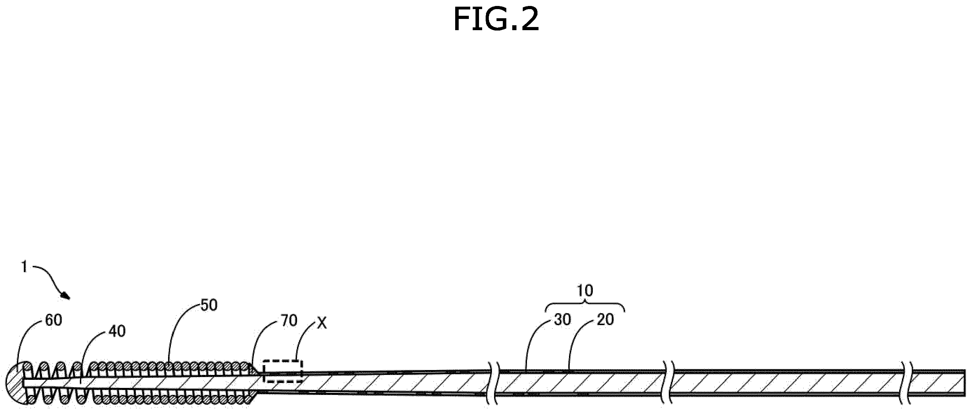

Fig. 1 is an explanatory diagram illustrating an entire configuration of a guide wire 1 according to a first embodiment. Fig. 2 is an explanatory diagram illustrating a cross-sectional configuration of the guide wire 1. Hereinafter, the left side in Fig. 1 is referred to as "distal end side" of the guide wire 1 and constituent members, and the right side in Fig. 1 is referred to as "proximal end side" of the guide wire 1 and constituent members. The distal end side of the guide wire 1 is a side to be inserted into a body (distal side), and the proximal end side of the guide wire 1 is a side operated by an operator such as a doctor (near side). The left-right direction in FIG. 1 is referred to as "stretching direction" or "axis direction" of the guide wire 1 and each constituent member. Fig. 2 illustrates a longitudinal section along the stretching direction of the guide wire 1. The guide wire 1 is a medical device used when a catheter is inserted into a blood vessel or a digestive organ, and includes a coating film 10, a core shaft 40, a coil body 50, a distal end side joint portion 60, and a proximal end side joint portion 70.

The core shaft 40 has an elongated shape, and the coating film 10 is formed on the outer surface of the core shaft 40. The coating film 10 includes a marker portion 20 and a resin portion 30. The coil body 50 is wound around an outer periphery of the distal end portion of the core shaft 40.

The core shaft 40 is an elongated (tapered) member configured so that an outer diameter decreases from the proximal end side to the distal end side. The core shaft 40 may be formed of a material such as a stainless alloy (SUS302, SUS304, SUS316, and the like), a superelastic alloy such as a Ni-Ti alloy, a piano wire, a nickel-chromium base alloy, a cobalt alloy, or tungsten. The core shaft 40 may be formed of a well-known material other than the materials mentioned above. A length of the core shaft 40 is not particularly limited, but a range of 1000 mm to 5000 mm may be used as an example. The outer diameter of the core shaft 40 is also not particularly limited, but a range of 0.1 mm to 1.0 mm may be used as an example.

The coil body 50 is configured by one or a plurality of coils, and is wound around the core shaft 40 to cover the outer periphery on the distal end side of the core shaft 40. Here, the coil body 50 is wound around a part of a small diameter portion and a tapered portion on the distal end side of the core shaft 40. The coil configuring the coil body 50 may be a single coil formed by spirally winding one wire having a circular cross section to form a cylindrical shape, or a hollow, twisted wire coil obtained by forming a twisted wire obtained by twisting a plurality of wires into a cylindrical shape. The coil body 50 may be configured by combining a single coil and a hollow twisted wire coil. The coil body 50 may be formed of, for example, a stainless alloy (SUS302, SUS304, SUS316, and the like), a superelastic alloy such as an Ni-Ti alloy, a piano wire, a nickel-chromium base alloy, a cobalt alloy, a radiolucent alloy such as tungsten, gold, platinum, tungsten, or a radiopaque alloy such as an alloy containing these elements (for example, a platinum-nickel alloy). The coil body 50 may be formed of a well-known material other than the materials mentioned above. A length of the coil body 50 is not particularly limited, but for example, 10 mm to 100 mm may be used as an example. An outer diameter of the coil body 50 is not particularly limited, but may be in a range of 0.1 mm to 1.0 mm, for example, and is configured to be constant from the distal end to the proximal end. The coil body 50 may include a loosely wound portion and a tightly wound portion having different coil pitches.

The distal end of the coil body 50 is joined to the distal end of the core shaft 40 by the distal end side joint portion 60. The proximal end of the coil body 50 is joined to the core shaft 40 by the proximal end side joint portion 70. The coil body 50 is fixed to the core shaft 40 by the distal end side joint portion 60 and the proximal end side joint portion 70. The distal end side joint portion 60 and the proximal end side joint portion 70 are formed of a metal solder such as silver solder, gold solder, zinc, a Sn-Ag alloy, or an Au-Sn alloy, and by such a metal solder, the coil body 50 and the core shaft 40 are adhered and fixed. It is noted that the distal end side joint portion 60 and the proximal end side joint portion 70 may be formed of an adhesive such as an epoxy adhesive, and by such an adhesive, the coil body 50 and the core shaft 40 may be adhered and fixed. The distal end side joint portion 60 and the proximal end side joint portion 70 may be formed of different materials.

The coating film 10 is formed on the outer surface of the core shaft 40, and includes the marker portion 20 and the resin portion 30. The coating film 10 is arranged on the proximal end side of the core shaft 40 relative to the proximal end side joint portion 70. In the coating film 10, a length of a zone where the marker portion 20 is formed (marker display zone) is not particularly limited, but a range of 100 mm to 500 mm may be used as an example. The coating film 10 may cover not only the outer surface of the core shaft 40 on the proximal end side relative to the proximal end side joint portion 70, but also the proximal end side joint portion 70, the coil body 50, and the distal end side joint portion 60.

The marker portion 20 contains a pigment and is formed on the outer surface of the core shaft 40. The marker portion 20 forms a part of the outer surface of the coating film 10. The marker portion 20 is a linear portion formed in a part of the coating film 10, and is configured to be visually distinguishable from the resin portion 30. When the guide wire 1 is observed from the outside, the marker portion 20 is seen as a linear pattern on the guide wire 1, and when the operator observes a change of an orientation and a position of the pattern when operating the guide wire 1, it is possible to confirm push-pull and rotation operations of the guide wire 1.

The marker portion 20 is formed to have a wavy pattern in a developed view obtained when the outer periphery of the core shaft 40 is expanded. That is, when the pigment dripping toward the core shaft 40 is moved along the stretching direction in a state where the core shaft 40 is reciprocated and rotated by a predetermined angle (for example, 180°) to the left and right with the stretching direction (axis direction) as a rotation axis, it is possible to form such a wavy pattern. The marker portion 20 may be spirally formed on the core shaft 40. That is, when the pigment dripping toward the core shaft 40 is moved along the stretching direction in a state where the core shaft 40 is rotated in one direction with the stretching direction (axis direction) as a rotation axis, it is possible to from a spiral pattern.

The pigment forming the marker portion 20 may be any one of an inorganic pigment and an organic pigment, but it is preferable to use an inorganic pigment in terms of heat resistance. As the inorganic pigment, it is possible to use carbon black, mica, titanium dioxide, titanium yellow, Prussian blue, Milori blue, cobalt blue, ultramarine, viridian, and the like. The marker portion 20 may contain a resin, and may contain PAI (polyamide-imide), PTFE (polytetrafluoroethylene), PVDF (polyvinylidene fluoride), PFA (perfluoroalkoxy alkane), FEP (perfluoroethylene propene), ETFE (ethylene tetrafluoroethylene), PE (polyethylene), and PP (polypropylene), for example. The marker portion 20 may be formed of a well-known material other than the above examples. The marker portion 20 may contain the same type of resin as or a type different from the resin forming the resin portion 30.

The resin portion 30 contains a resin and is formed on the outer surface of the core shaft 40. The resin portion 30 forms a part of the outer surface of the coating film 10.

The resin portion 30 may be formed of PAI (polyamide-imide), PTFE (polytetrafluoroethylene), PVDF (polyvinylidene fluoride), PFA (perfluoroalkoxy alkane), FEP (perfluoroethylene propene), ETFE (ethylene tetrafluoroethylene), PE (polyethylene), and PP (polypropylene), for example. The resin portion 30 may be formed of a well-known material other than the above examples. The resin portion 30 may contain a pigment, which may be any one of an inorganic pigment and an organic pigment, but it is preferable to use the inorganic pigment in terms of heat resistance. As the inorganic pigment, it is possible to use carbon black, mica, titanium dioxide, titanium yellow, Prussian blue, Milori blue, cobalt blue, ultramarine, viridian, and the like. When the resin portion 30 is formed to be different in color from the marker portion 20, it is possible to increase the visibility of the marker portion 20.

The marker portion 20 and the resin portion 30 are repeatedly represented as a result of the marker portion 20 and the resin portion 30 being alternately and continuously present without gaps along the stretching direction of the guide wire 1.

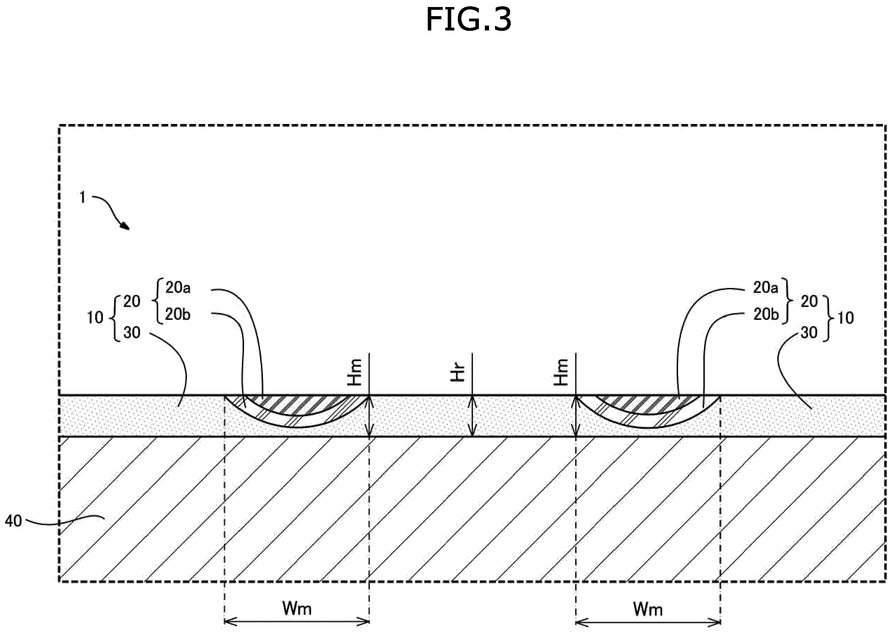

Fig. 3 is an explanatory diagram obtained when a detail X of Fig. 2 is enlarged. The detail X is a portion located on the proximal end side relative to the proximal end side joint portion 70 in Fig. 2 and provided with the coating film 10 on the outer surface of the core shaft 40, and is provided with the marker portion 20. The X portion is used as an example to describe a detailed configuration of the marker portion 20 and the resin portion 30.

A thickness of a portion of the coating film 10 where the marker portion 20 is represented on the outer surface is defined as Hm. It is assumed here that the thickness Hm is a maximum value of a length from the outer surface of the marker portion 20 to the outer surface of the core shaft 40 in the longitudinal section of the core shaft 40. The thickness Hm is not particularly limited, but a range of 0.01 mm to 0.1 mm may be used as an example. In Fig. 3, the outer surface of the marker portion 20 is flat, and thus, the thickness Hm remains substantially the same at any point in the stretching direction of the core shaft 40.

In the longitudinal section of the core shaft 40, a thickness of a portion of the coating film 10 in which the resin portion 30 is represented on the outer surface, the portion existing between adjacent ones of the marker portions 20, is defined as Hr. It is assumed here that the thickness Hr is a maximum value of a length from the outer surface of the resin portion 30 to the outer surface of the core shaft 40 in the longitudinal section of the core shaft 40. The thickness Hr is not particularly limited, but a range of 0.01 mm to 0.1 mm may be used as an example. In Fig. 3, the outer surface of the resin portion 30 is flat, and thus, the thickness Hr remains substantially the same at any point in the stretching direction of the core shaft 40.

The thickness Hm of the portion of the coating film 10 where the marker portion 20 is represented on the outer surface is substantially the same as the thickness Hr of the portion of the coating film 10 where the resin portion 30 is represented on the outer surface. In other words, the outer surface of the marker portion 20 and the outer surface of the resin portion 30 are formed to be flat. For example, it is preferable that a difference between the thickness Hm and the thickness Hr is equal to or less than 20 µm, and more preferably equal to or less than 10 µm.

In the longitudinal section of the core shaft 40, a length of the marker portion 20 in the stretching direction of the core shaft 40 is defined as a cross-sectional width of the marker portion 20. Further, the cross-sectional width of the marker portion 20 on the outer surface of the marker portion 20 is defined as an outer cross-sectional width Wm. The marker portion 20 is of arc shape, and of convex shape inward in a radial direction of the core shaft 40, and thus, the cross-sectional width of the marker portion 20 gradually decreases inward in the radial direction of the core shaft 40. In other words, the cross-sectional width of the marker portion 20 gradually increases outward in the radial direction of the core shaft 40.

The marker portion 20 includes a first region 20a including the outer surface of the marker portion 20 and a second region 20b provided around the first region 20a. In the longitudinal section of the core shaft 40, the first region 20a is of arc shape, and is of convex shape toward the inside of the core shaft 40 in the radial direction. The second region 20b is of arc shape to cover a surrounding area of the first region 20a, and is of convex shape toward the inside of the core shaft 40 in the radial direction. Here, a content of the pigment contained in the second region 20b is smaller than a content of the pigment contained in the first region 20a. If the marker portion 20 consists of the resin and the pigment, the content of the pigment contained in the second region 20b is larger than the content of the pigment contained in the first region 20a.

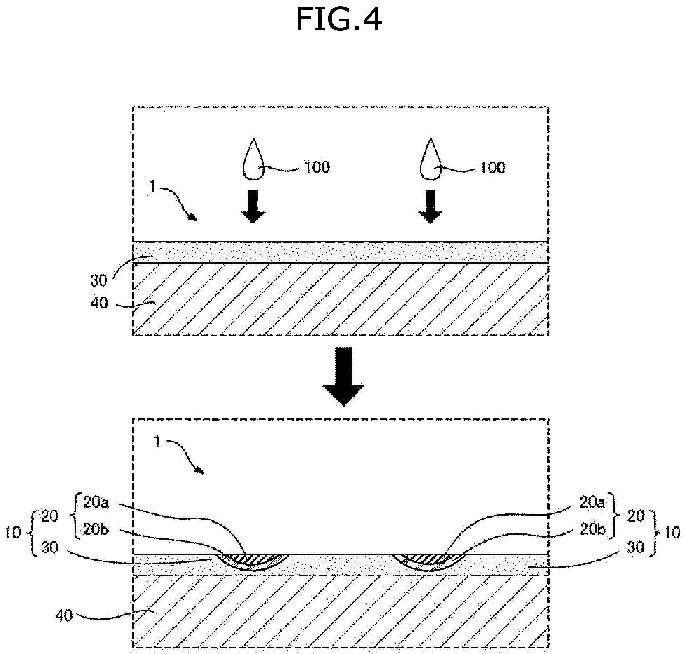

Fig. 4 is an explanatory diagram illustrating a method of manufacturing the guide wire 1. If an average particle size of a pigment 100 contained in the marker portion 20 is smaller than an average particle size of the resin of the resin portion 30, it is possible to manufacture the marker portion 20 represented by a wavy pattern on the resin portion 30 according to the following steps, for example. Firstly, the resin portion 30 is coated on the outer surface of the core shaft 40. Next, in a state where the core shaft 40 is reciprocated and rotated by a predetermined angle (for example, 180°) to the left and right with the stretching direction (axis direction) as a rotation axis, the pigment 100 dripping toward the core shaft 40 is moved along the stretching direction. As a result, the pigment 100 is applied in a wavy pattern on the outer surface of the resin portion 30. Next, when the pigment 100 is heated with a heater or the like, particles of the pigment 100 enter between particles of the resin portion 30. Finally, when the pigment 100 and the resin portion 30 are dried, the pigment 100 and the resin portion 30 are fixed, and the coating film 10 is formed. According to this method, the marker portion 20 is formed in an arc shape to be convex toward the inside of the core shaft 40 in the radial direction around a point where the pigment 100 is applied. As a result, in the marker portion 20, in an area near the point where the pigment 100 is applied, a content of the pigment 100 is large, and the content of the pigment 100 gradually decreases as a distance from the point where the pigment 100 is applied increases. According to such a procedure, it is possible to form the first region 20a and the second region 20b.

According to the configuration of the present embodiment, when the guide wire 1 is provided with the marker portion 20, it is possible to suppress an increase of the outer diameter of the guide wire 1. When the increase of the outer diameter of the guide wire 1 is suppressed, the operator is capable of selecting a device having a smaller inner diameter of a lumen in using a device used with the guide wire such as a catheter having a lumen through which the guide wire is inserted. It is also easier for the operator to insert the guide wire into a site having a small diameter such as a peripheral blood vessel.

According to the configuration of the present embodiment, as compared to a case where the cross-sectional width of the marker portion 20 is substantially constant toward the inside of the core shaft 40 in the radial direction, it is possible to increase a contact area between the marker portion 20 and the resin portion 30, and thus, it is possible to decrease a possibility that the marker portion 20 and the resin portion 30 peel off. As a result, it is possible to prevent the marker portion 20 from peeling off and remaining in a body of a patient or in the device used with the guide wire such as a catheter.

According to the configuration of the present embodiment, when the content of pigment contained in a marker portion 20b contacting the resin portion 30 is reduced, it is possible to maintain adhesion between the marker portion 20b and the resin portion 30 and it is possible to decrease a possibility that the marker portion 20b and the resin portion 30 peel off.

### <Second Embodiment>

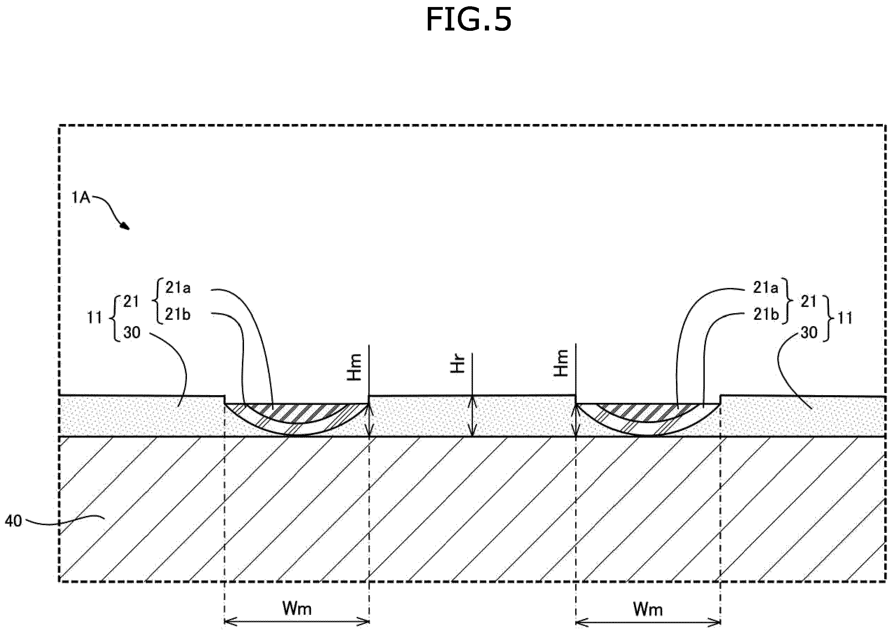

Fig. 5 is an explanatory diagram illustrating a cross-sectional configuration of a guide wire 1A according to a second embodiment. The guide wire 1A includes a coating film 11, and the coating film 11 includes a marker portion 21 instead of the marker portion 20 of the first embodiment in Fig. 3. The thickness Hm of the portion of the coating film 11 where the marker portion 21 is represented on the outer surface is smaller than the thickness Hr of the portion of the coating film 11 where the resin portion 30 is represented on the outer surface. In other words, the outer surface of the marker portion 21 is radially inside the core shaft 40 relative to the outer surface of the resin portion 30.

According to the configuration of the present embodiment, the outer surface of the marker portion 21 does not easily contact an inner peripheral surface of a device used with the guide wire such as a catheter (not illustrated) or a wall surface (not illustrated) of an internal organ or a blood vessel of a body, and it is possible to decrease a possibility that the marker portion 21 peels off. A contact of an inner peripheral surface of the device used with the guide wire such as a catheter (not illustrated) and a wall surface (not illustrated) of an internal organ, a blood vessel, or the like of a body, with the outer surface of the marker portion 21 is not easy, but a contact thereof with the outer surface of the resin portion 30 is easier, and thus, it is possible to improve slidability of the guide wire 1A.

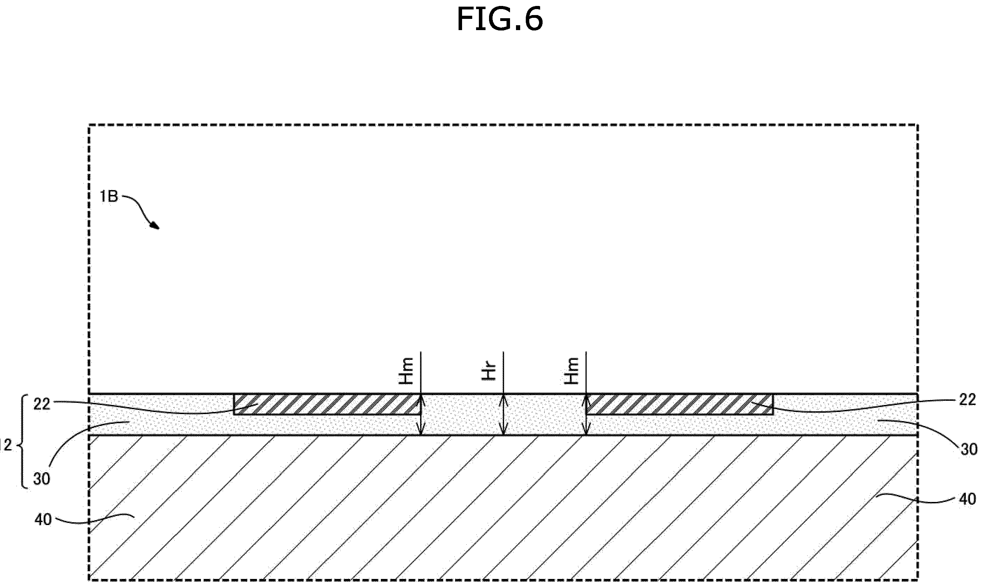

Fig. 6 is an explanatory diagram illustrating a cross-sectional configuration of a guide wire 1B according to a third embodiment. The guide wire 1B includes a coating film 12, and the coating film 12 includes a marker portion 22 instead of the marker portion 20 of the first embodiment in Fig. 3. In the marker portion 22, a cross-sectional width of the marker portion 22 is substantially constant in a radial direction of the core shaft 40. The marker portion 22 has a substantially constant overall pigment content over the longitudinal section of the marker portion 22. For example, the coating film 12 covers the outer surface of the core shaft 40 with the resin portion 30 having a groove formed in a wavy shape or a spiral shape toward the stretching direction of the core shaft 40, and may be formed by fitting the linearly formed marker portion 22 into the groove formed in the resin portion 30.

According to the configuration of the present embodiment, as compared to a case where the cross-sectional width of the marker portion decreases toward the inside of the core shaft 40 in the radial direction, it is possible to increase a thickness of an end portion of the marker portion 22, and thus, it is possible to prevent the marker portion 22 from being damaged starting from the end portion of the marker portion 22.

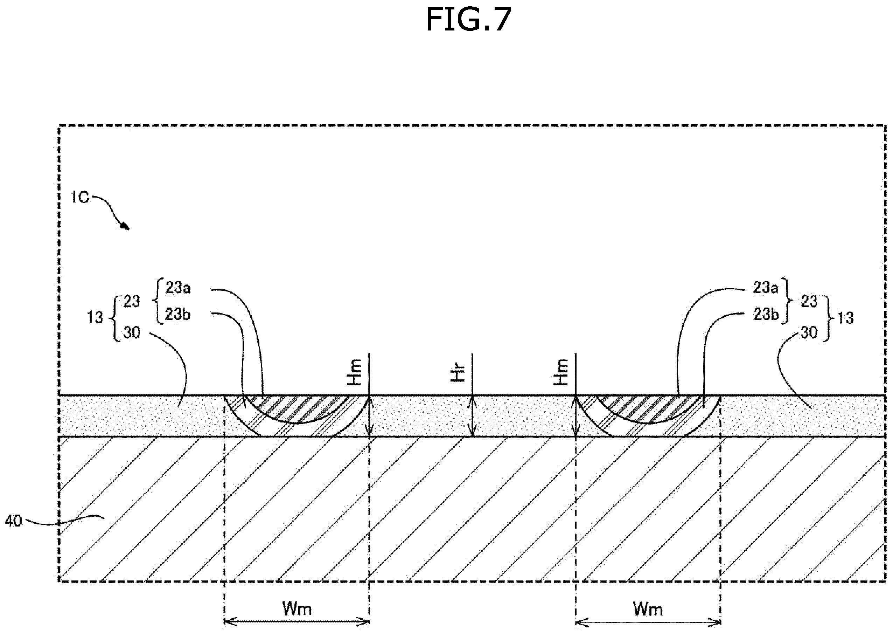

Fig. 7 is an explanatory diagram illustrating a cross-sectional configuration of a guide wire 1C according to a fourth embodiment. The guide wire 1C includes a coating film 13, and the coating film 13 includes a marker portion 23 instead of the marker portion 20 of the first embodiment in Fig. 3. An inner surface of the marker portion 23 contacts the outer surface of the core shaft 40. In this case, a maximum width of the marker portion 23 and the thickness Hm of a portion of the coating film 13 where the marker portion 23 is represented on the outer surface are equal.

According to the configuration of the present embodiment, as compared to a case where the inner surface of the marker portion does not contact the outer surface of the core shaft 40, it is possible to increase a thickness of the marker portion 23, and thus, a strength of the marker portion 23 is improved.

### <Modifications of Embodiment>

The present invention is not limited to the above-described embodiments, and may be implemented in various modes without departing from the scope of the present invention. The following modifications can be applied, for example.

### [First Modification]

It is assumed that the marker portions 20, 21, 22, and 23 (see Figs. 3, 5, 6, and 7) of the first to fourth embodiments are displayed in a part (marker display zone) of the core shaft 40. However, the marker portions 20, 21, 22, and 23 may be formed over the entire core shaft 40. The marker portions 20, 21, 22, and 23 are assumed to be drawn with one pattern. However, the marker portions 20, 21, 22, and 23 may have a plurality of types of patterns. In this case, the marker portions 20, 21, 22, and 23 may have a mode in which the pattern changes continuously, or may have a different pattern for each predetermined zone. The marker portions 20, 21, 22, and 23 may be cut somewhere in the core shaft 40 and drawn at a plurality of locations. For example, in the marker portions 20, 21, 22, and 23, a plurality of types of patterns may be drawn at a plurality of locations at predetermined intervals, or an annular pattern may be continuously drawn at equal intervals.

### [Second Modification]

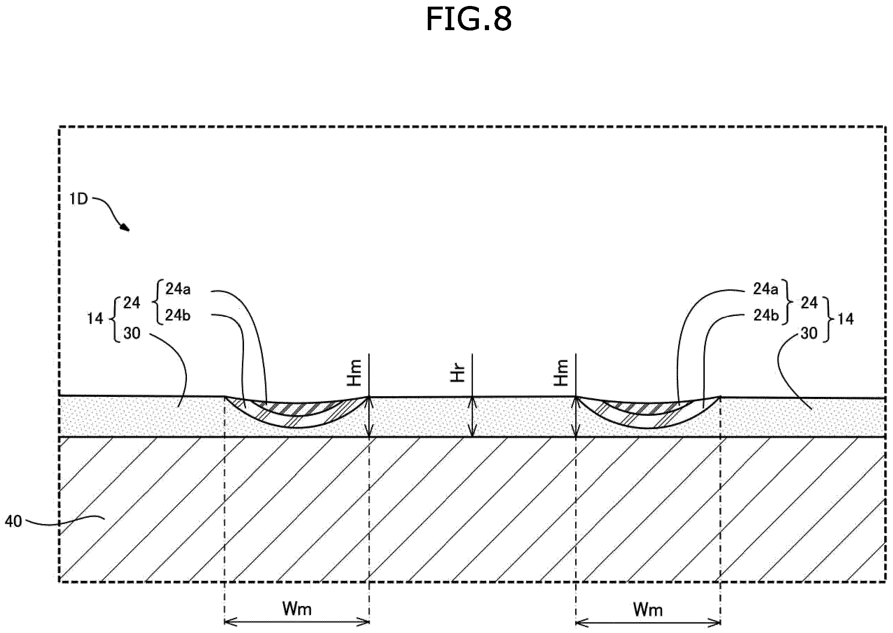

Fig. 8 illustrates a guide wire 1D which is a modification of the first to fourth embodiments. The guide wire 1D includes a coating film 14, and the coating film 14 includes a marker portion 24. An outer surface of the marker portion 24 is of arc shape and is recessed toward the inside of the core shaft 40 in the radial direction. Therefore, the thickness Hm of the portion of the coating film 14 where the marker portion 24 is represented on the outer surface is not substantially constant in the stretching direction of the core shaft 40. In the case of Fig. 8, the thickness of the end portion of the marker portion 24 is a maximum value of the thickness of the portion of the coating film 14 where the marker portion 24 is represented on the outer surface. In the marker portions 20, 21, 22, and 23 according to the first to fourth embodiments, in the longitudinal section of the core shaft 40, similarly to the marker portion 24, the outer surface of the marker portions 20, 21, 22, and 23 may be recessed toward the inside of the core shaft 40 in the radial direction. As long as the outer surface of the marker portions 20, 21, 22, and 23 does not protrude outward in the radial direction of the core shaft 40 from the outer surface of the resin, the outer surface of the marker portions 20, 21, 22, and 23 may be recessed outward in the radial direction of the core shaft 40.

### [Third Modification]

The guide wires 1, 1A, 1B, and 1C of the first to fourth embodiments are assumed to include the coil body 50 at the distal end. However, it is not required that the guide wires 1, 1A, 1B, and 1C include the coil body 50. In this case, the coating films 10, 11, 12, and 13 may be provided at the distal end portion of the core shaft 40.

Although the aspects have been described based on the embodiments and the modifications, the embodiments of the above-described aspects are for facilitating understanding of the aspects, and do not limit the aspects. The aspects can be modified and improved without departing from the scope of the claims.

### DESCRIPTION OF REFERENCE NUMERALS

- 1, 1A, 1B, 1C, 1D: Guide wire
- 10, 11, 12, 13, 14: Coating film
- 20, 21, 22, 23, 24: Marker portion
- 20a: first region of marker portion
- 20b: second region of marker portion
- 30: Resin portion
- 40: Core shaft
- 50: Coil body
- 60: Distal end side joint portion
- 70: Proximal end side joint portion
- 100: Pigment
- Hm: Thickness of portion where marker portion is represented on outer surface
- Hr: Thickness of portion where resin portion is represented on outer surface
- Wm: Outer cross-sectional width of marker portion

## Claims

1. A guide wire (1, 1A, 1C, 1D) comprising:
a core shaft (40) having an elongated outer shape; and
a coating film (10, 11, 13, 14) formed on an outer surface of the core shaft (40), the coating film (10, 11, 13, 14) containing a resin,
wherein the coating film (10, 11, 13, 14) includes a marker portion (20, 21, 23, 24) that contains a pigment linearly represented on an outer surface of the coating film (10, 11, 13, 14) and a resin portion (30) that does not contain the pigment,
wherein, on the outer surface of the coating film (10, 11, 13, 14), the marker portion (20, 21, 23, 24) and the resin portion (30) are alternately represented in a stretching direction, and
wherein a thickness of a portion of the coating film (10, 11, 13, 14) where the marker portion (20, 21, 23, 24) is represented on the outer surface and a thickness of a portion of the coating film (10, 11, 13, 14) where the resin portion (30) is represented on the outer surface are substantially the same, or the thickness of the portion of the coating film (10, 11, 13, 14) where the marker portion (20, 21, 23, 24) is represented is smaller than the thickness of the portion of the coating film (10, 11, 13, 14) where the resin portion (30) is represented, and
wherein in a longitudinal section along a stretching direction of the core shaft (40), a cross-sectional width of the marker portion (20, 21, 23, 24) decreases toward an inside of the core shaft (40) in a radial direction.

2. The guide wire according to claim 1, wherein the marker portion (20, 21, 23, 24) includes a first region (20a) including the outer surface of the marker portion (20, 21, 23, 24) and a second region (20b) provided around the first region (20a), and a content of the pigment contained in the second region (20b) is smaller than a content of the pigment contained in the first region (20a).

3. The guide wire according to claims 1 or 2, wherein an average particle size of the pigment contained in the marker portion (20, 21, 23, 24) is smaller than an average particle size of the resin portion (30).

## Patentansprüche

1. Führungsdraht (1, 1A, 1C, 1D) mit:
einem Kernschaft (40), der eine langgestreckte Außenform aufweist, und
einem Beschichtungsfilm (10, 11, 13, 14), der auf einer Außenfläche des Kernschafts (40) gebildet ist, wobei der Beschichtungsfilm (10, 11, 13, 14) ein Harz enthält,
wobei der Beschichtungsfilm (10, 11, 13, 14) einen Markierungsabschnitt (20, 21, 23, 24), der einen auf einer Außenfläche des Beschichtungsfilms (10, 11, 13, 14) linear dargestellten Farbstoff enthält, und einen Harzabschnitt (30) aufweist, der den Farbstoff nicht enthält,
wobei der Markierungsabschnitt (20, 21, 23, 24) und der Harzabschnitt (30) auf der Außenfläche des Beschichtungsfilms (10, 11, 13, 14) abwechselnd in einer Dehnungsrichtung dargestellt sind und
wobei eine Dicke eines Abschnitts des Beschichtungsfilms (10, 11, 13, 14), in dem der Markierungsabschnitt (20, 21, 23, 24) auf der Außenfläche dargestellt ist, und eine Dicke eines Abschnitts des Beschichtungsfilms (10, 11, 13, 14), in dem der Harzabschnitt (30) auf der Außenfläche dargestellt ist, im Wesentlichen gleich sind, oder die Dicke des Abschnitts des Beschichtungsfilms (10, 11, 13, 14), in dem der Markierungsabschnitt (20, 21, 23, 24) dargestellt ist, geringer als die Dicke des Abschnitts des Beschichtungsfilms (10, 11, 13, 14) ist, in dem der Harzabschnitt (30) dargestellt ist, und
wobei in einem Längsschnitt entlang einer Dehnungsrichtung des Kernschafts (40) eine Querschnittsbreite des Markierungsabschnitts (20, 21, 23, 24) zu einer Innenseite des Kernschafts (40) hin in einer Radialrichtung abnimmt.

2. Führungsdraht nach Anspruch 1, bei dem der Markierungsabschnitt (20, 21, 23, 24) einen ersten Bereich (20a), der die Außenfläche des Markierungsabschnitts (20, 21, 23, 24) aufweist, und einen zweiten Bereich (20b) aufweist, der um den ersten Bereich (20a) herum vorgesehen ist, und ein Gehalt an dem im zweiten Bereich (20b) enthaltenen Farbstoff geringer als ein Gehalt an dem im ersten Bereich (20a) enthaltenen Farbstoff ist.

3. Führungsdraht nach Anspruch 1 oder 2, bei dem eine mittlere Teilchengröße des in dem Markierungsabschnitt (20, 21, 23, 24) enthaltenen Farbstoffs kleiner als eine mittlere Teilchengröße des Harzabschnitts (30) ist.

## Revendications

1. Fil de guidage (1, 1A, 1C, 1D) comprenant :
une tige centrale (40) présentant une forme extérieure allongée, et
un film de revêtement (10, 11, 13, 14) réalisé sur une surface extérieure de la tige centrale (40), le film de revêtement (10, 11, 13, 14) contenant une résine,
le film de revêtement (10, 11, 13, 14) comprenant une partie de marquage (20, 21, 23, 24) qui contient un colorant représenté de manière linéaire sur une surface extérieure du film de revêtement (10, 11, 13, 14), et une partie en résine (30) qui ne contient pas le colorant,
la partie de marquage (20, 21, 23, 24) et la partie en résine (30) étant représentées en alternance dans un sens d'extension sur la surface extérieure du film de revêtement (10, 11, 13, 14), et
une épaisseur d'une partie du film de revêtement (10, 11, 13, 14) dans laquelle la partie de marquage (20, 21, 23, 24) est représentée sur la surface extérieure et une épaisseur d'une partie du film de revêtement (10, 11, 13, 14) dans laquelle la partie en résine (30) est représentée sur la surface extérieure étant sensiblement égales, ou l'épaisseur de la partie du film de revêtement (10, 11, 13, 14) dans laquelle la partie de marquage (20, 21, 23, 24) est représentée étant inférieure à l'épaisseur de la partie du film de revêtement (10, 11, 13, 14) dans laquelle la partie en résine (30) est représentée, et
une largeur en coupe transversale de la partie de marquage (20, 21, 23, 24), dans une coupe longitudinale le long d'un sens d'extension de la tige centrale (40), diminuant dans un sens radial vers un côté intérieur de la tige centrale (40).

2. Fil de guidage selon la revendication 1, la partie de marquage (20, 21, 23, 24) comprenant une première zone (20a) qui comprend la surface extérieure de la partie de marquage (20, 21, 23, 24), et une deuxième zone (20b) prévue autour de la première zone (20a), et une teneur en colorant contenue dans la deuxième zone (20b) étant inférieure à une teneur en colorant contenue dans la première zone (20a).

3. Fil de guidage selon la revendication 1 ou 2, une taille moyenne des particules du colorant contenu dans la partie de marquage (20, 21, 23, 24) étant inférieure à une taille moyenne des particules de la partie en résine (30).
